# EUROPEAN PATENT SPECIFICATION

(11) **EP 3 756 692 B1**
(45) Date of publication and mention of the grant of the patent: **19.07.2023**
(21) Application number: 19754263.2
(22) Date of filing: 18.02.2019
(51) Int. Cl.: A61L 2/10, A61L 2/26

(54) **ULTRAVIOLET CURVED TYPE STERILIZER**
GEKRÜMMTER ULTRAVIOLETT-STERILISATOR
STÉRILISATEUR DE TYPE INCURVÉ AUX ULTRAVIOLETS

(30) Priority: 19.02.2018 KR 20180019417
(43) Date of publication of application: 30.12.2020
(73) Proprietor: FHI Korea Co. Ltd., Seocho-gu, Seoul 06119 (KR)
(72) Inventor: SHIN, Sang Yong, Youngin-Si Gyeonggi-do 16851 (KR); VACH, Young Woo, Wonju-Si Gangwon-do 26388 (KR)
(74) Representative: LLR
(86) International application number: PCT/KR2019/001897
(87) International publication number: WO 2019/160382

(56) References cited:
- JP-B1- 5 989 854
- KR-A- 20110 087 364
- KR-A- 20150 012 971
- KR-A- 20150 012 971
- KR-A- 20170 009 390
- KR-A- 20170 028 472
- KR-B1- 100 838 348
- US-A1- 2006 284 109
- US-A1- 2014 158 917

## Description

### Technical Field

The present invention relates to a ultraviolet curved type sterilizer and, more particularly, to a ultraviolet curved type sterilizer that is configured to increase a sterilization effect by projecting ultraviolet rays to be overlapped in all directions of 360 degree angles on a specific area.

### Background Art

Recently, generation of various pollutions such as fine dust, automobile exhaust gas, dust, and the like has increased. People are exposed to these various pollutions and are easily infected with diseases. Most people with such diseases go to hospitals to treat the diseases.

However, as many patients come to the hospitals for examination and treatment, viruses are unintentionally spread to medical treatment equipment and public objects used by the patients.

As a result, patients are secondarily exposed to an environment of virus infection at the hospital visited for treatment of their diseases.

In order to solve this problem, development of a device for sterilizing medical equipment and objects installed in a hospital space has been actively conducted.

Examples of portable ultraviolet sterilizers are disclosed in : US2014158917, US2006284109 and KR20150012971.

However, currently developed ultraviolet sterilizers, including mobile ultraviolet sterilizers, may sterilize only some areas during being moved. In addition, such an ultraviolet sterilizer has a problem that an angle of ultraviolet projection is not wide.

In addition, safety accidents are often caused by the use of harmful chemicals for the cleanliness of public places, such as public toilets, damp basements, and the like.

In order to solve this problem, a device capable of maintaining cleanliness by using the ultraviolet sterilizer instead of chemicals is needed.

### Disclosure

### Technical Problem

Accordingly, the present invention has been made keeping in mind the above problems occurring in the prior art, and an objective of the present invention is to provide a ultraviolet curved type sterilizer that can project ultraviolet rays on a wide area (360 degree angle directions and ceiling and floor surfaces) and allows the ultraviolet rays to be overlapped in all directions for some areas using optical properties of light, wherein the some areas are places where objects and medical equipment that are contaminated are most contacted by human hands. Accordingly, the sterilization effect for the some areas can be increased.

The technical problem of the present invention is not limited to the problems mentioned above, and other technical problems not mentioned will be clearly understood by those skilled in the art from the following description.

### Technical Solution

In order to accomplish the above object, the present invention may provide a ultraviolet curved type sterilizer, the sterilizer including: a base portion having a space accommodating a power supply device therein and a support portion formed on a top end portion thereof in a perpendicular direction to a bottom floor;

a plurality of curved central reflection portions each fixed to the support portion by having one end and an opposite end inclined at the same angle from the support portion taking a middle of length as a center;

a central ultraviolet lamp installed to project ultraviolet rays to the curved central reflection portion toward an outer side, the central ultraviolet lamp being formed of a quartz glass material;

an upper end reflection portion formed with an accommodating space and fixedly installed at a top end of the support portion; and

an upper end reflection portion formed with an accommodating space and fixedly installed at a top end of the support portion; and

an upper end ultraviolet lamp installed in an accommodating space of the upper end reflection portion and configured to project the ultraviolet rays toward an outer side, wherein the sterilizer comprises holder angles and holders installed at each of the opposite ends of the central ultraviolet lamp, each holder angle having one side connected to one of the holders and an opposite side connected to one end of the opposite ends of the curved central reflection portion, thereby allowing the holders to be angle- adjusted from the curved central reflection portion, and when the central ultraviolet lamp is assembled to the holder angle, the central ultraviolet lamp is able to be used by being modified in a '<' shape or a '>' shape.

The plurality of curved central reflection portions may be installed at regular intervals on the support portion so that the ultraviolet rays projected by the central ultraviolet lamp installed on an outer side are allowed to be partially overlapped.

The curved central reflection portion may be inclined at an acute angle from the support portion, the central ultraviolet lamp corresponds to the inclination of the curved central reflection portion, and the central ultraviolet lamp has the shape of a straight type or a curved type. The base portion may be provided with motion detection sensors on a side surface thereof at regular intervals and configured to detect a movement of an object, and each of the base portion and the support portion includes a vent configured to dissipate heat generated from the power supply device to the outside.

The motion detection sensors may be connected to the power supply device, so the sterilizer enables the power supply device to be turned off when the motion detection sensors detect a motion and enables the power supply device to be turned on even when the motion detection sensors are off.

### Advantageous Effects

As described above, a ultraviolet curved type sterilizer according to the present invention can effectively sterilize a wide area, thereby blocking secondary infection in a short time. In addition, there is a 360 degree omnidirectional sterilization effect in areas where germs may be easily spread and in areas including corners or wet places. Especially, for the areas where a person's hand touches, the sterilization effect can be maximized by overlapped ultraviolet projection.

In addition, the ultraviolet curved type sterilizer of the present invention can exert an optimal sterilization effect by adjusting the intensity depending on the germs to be killed and automatically stop ultraviolet projection to prevent human exposure to strong ultraviolet rays when a person approaches.

### Description of Drawings

FIG. 1 is a perspective view of a ultraviolet curved type sterilizer according to an embodiment of the present invention.
FIG. 2 is an exploded perspective view of the ultraviolet curved type sterilizer of FIG. 1.
FIG. 3 is an operation diagram showing an operation state of a holder angle of FIG. 2.
FIG. 4 shows views illustrating areas on which ultraviolet rays overlap according to the operation of the ultraviolet curved type sterilizer of FIG. 1.
FIG. 5 shows perspective views of ultraviolet curved type sterilizers according to other embodiments of the present invention.
FIG. 6 shows photographs illustrating a front surface state and a top end surface state of FIG. 5(d).
FIG. 7 shows views illustrating overlapped distribution states of ultraviolet rays projected from the ultraviolet curved type sterilizer of FIG. 5.
FIG. 8 is data showing a projection amount of the ultraviolet curved type sterilizer of the present invention and a projection amount of a conventional ultraviolet sterilizer.
FIG. 9 is material data demonstrating the sterilization performance of the ultraviolet curved type sterilizer of the present invention.

### Best Mode

Advantages and features of the present invention and methods for achieving same will be clarified with reference to embodiments described below in detail together with the accompanying drawings. However, the present invention is not limited to the embodiments disclosed below, but may be implemented in various different forms.

However, the present embodiments are provided only to make the disclosure of the present invention complete and to fully inform the scope of the present invention to a person of ordinary skill in the art to which the present invention belongs, and the present invention may only be defined by the claims.

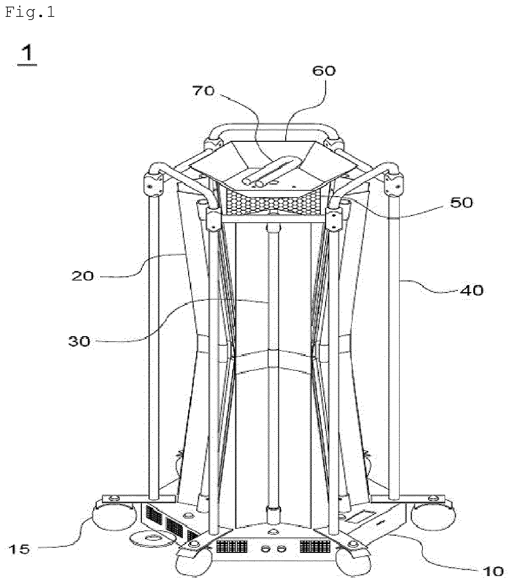

Hereinafter, a ultraviolet curved type sterilizer according to an embodiment of the present invention will be described in general with reference to FIG. 1.

FIG. 1 is a perspective view of a ultraviolet curved type sterilizer for an embodiment of the present invention. The ultraviolet curved type sterilizer 1 of the present invention may become a form in which the central ultraviolet lamp 30 is applied by being formed in a straight or curved shape.

In the ultraviolet curved type sterilizer 1 according to an embodiment of the present invention, a curved central reflection portion 20 is installed on a support portion 12, the central ultraviolet lamp 30 is installed on the curved central reflection portion 20, and an upper end ultraviolet lamp 70 is installed on a top end of the support portion, so the ultraviolet curved type sterilizer 1 may realize desired sterilization effect by projecting the ultraviolet rays in a manner of overlapping in top, bottom, left and right directions with the ultraviolet curved type sterilizer 1 as a center.

Particularly, as the central reflecting portion 20 is formed to have one end and an opposite end that are curved at an acute angle taking a middle of the length as a reference, the ultraviolet curved type sterilizer 1 of the present invention projects ultraviolet rays emitted from the installed central ultraviolet lamp 30 to be overlapped at a wide angle. In addition, six central ultraviolet lamps projecting the ultraviolet rays at a wide angle project the ultraviolet rays to be overlapped in all directions of 360 degree angles at the curved central reflection portions 20, respectively, due to optical properties of light.

Due to this structure, the ultraviolet curved type sterilizer 1 may increase sterilizing power of the portion where the ultraviolet rays are overlapped. In particular, being designed to overlap the ultraviolet rays on the portion being frequently touched by human hands, the ultraviolet curved type sterilizer is configured to perform projection and sterilization up to the bottom surface in all directions of 360 degree angles. Accordingly, the sterilization efficiency may be maximized.

In addition, the ultraviolet curved type sterilizer 1 of the present invention includes a controller 18 connected to a power supply device 11, whereby the central ultraviolet lamp 30 and the upper end ultraviolet lamp 70 may emit ultraviolet rays of various intensities under the control of the controller 18.

Therefore, the ultraviolet curved type sterilizer 1 may effectively sterilize a portion to be sterilized by adjusting the ultraviolet intensity in correspondence to the sterilization area and the available ultraviolet projection time.

In addition, the ultraviolet curved type sterilizer 1 includes motion detection sensors 13 that operate interlocked with the power supply device to detect the movement of objects, thereby, in correspondence to the presence or absence of detection function use of the motion detection sensors 13, allowing the power supply device 11 to be independently operated and also controlling the emission of ultraviolet rays.

The ultraviolet curved type sterilizer 1 may increase the sterilization effect without harming the human body due to the feature of controlling the intensity of ultraviolet rays and the feature of controlling whether ultraviolet rays are emitted.

Such a ultraviolet curved type sterilizer 1 includes a base portion 10, the curved central reflection portion 20, the central ultraviolet lamp 30, an upper end reflection portion 60, and the upper end ultraviolet lamp 70.

In addition, the ultraviolet curved type sterilizer 1 may include: a wheel 15 detachably installed on the base portion 10 by using a wheel bar 151 and a bolt 152; the motion detection sensors 13 configured to detect movement of objects; an emergency indicator light 16 configured to indicate a lamp life emergency state; and the like. Further, the ultraviolet curved type sterilizer 1 may also include a mesh net cylinder 50 allowing the support portion 12 of the base portion 10 not to be exposed and a handle portion 40 allowing a user to grip and move.

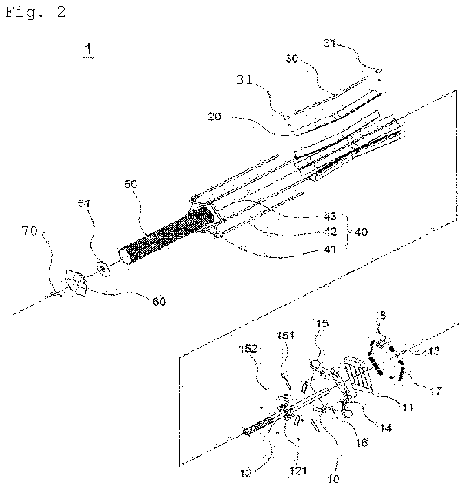

Hereinafter, with reference to FIGS. 2 to 4, components included in the ultraviolet curved type sterilizer 1 and operation of the ultraviolet curved type sterilizer 1 will be described in detail.

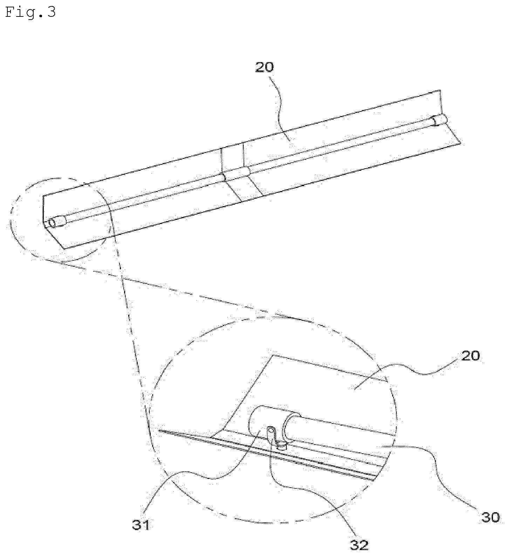

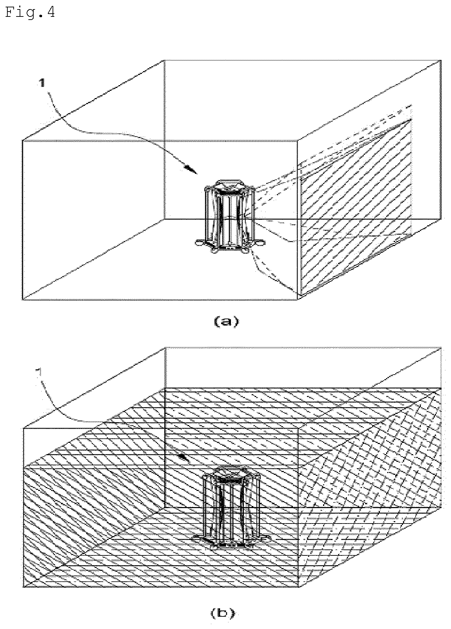

FIG. 2 is an exploded perspective view of the ultraviolet curved type sterilizer of FIG. 1, FIG. 3 is an operation diagram showing an operation state of a holder angle of FIG. 2, FIG. 4(a) is a view showing an area in which ultraviolet rays of one lamp overlap according to the operation of the ultraviolet curved type sterilizer of FIG. 1, and FIG. 4(b) is a view showing areas on which ultraviolet rays of all lamps overlap according to the operation of the ultraviolet curved type sterilizer of FIG. 1.

The base portion 10 serves as a pedestal of the ultraviolet curved type sterilizer 1. Here, the base portion 10 includes the power supply device 11 installed in an interior space and may include the controller 18 installed on the outer surface.

In addition, the base portion 10 may include the emergency indicator light 16 on a top end surface thereof indicating the emergency state in relation to the life of the power supply device 11 and the central ultraviolet lamp 30.

The controller 18 may control the operation of the power supply device 11, by a user and settings by being connected to the power supply device 11. In one example, the controller 18 may transmit signals different from each other to the power supply device 11 by the user's operation and control the operation of the central ultraviolet lamp 30 or the upper end ultraviolet lamp 70. In addition, when stable power supply is not possible due to a problem occurring in the power supply device 11, the controller 18 transmits a signal to the emergency indicator light 16, thereby turning on the emergency indicator light 16. Accordingly, the user may be alerted to recognize that there is a problem with the power supply device 11 or the central ultraviolet lamp 30.

In addition, the base portion 10 includes a support portion 12 installed on a top end portion in a direction perpendicular to a bottom surface, and includes vents 14 perforated at the side surface portions or the bottom end portions. At this time, the vents 14 are perforated in a quadrangle shape on the side surface portions, so that air may be entered from the outside or heat generated by the power supply device 11 may be discharged to the outside. In addition, a vent net 17 is installed in the vent 14, and foreign substance may be prevented from being introduced into an interior portion, that is, the power supply device 11, of the base portion 10 from the outside by the vent net 17.

In addition, the support portion 12 installed on a center of the top end surface of the base portion 10 is formed in a cylindrical shape. A plurality of curved central reflection portions 20 is installed on an outer circumferential surface of the support portion 12.

A support bar 121 may be formed on the support portion 12 protruding in a direction perpendicular to the support portion 12, that is, in a direction parallel to the base portion 10.

A plurality of the support bars 121 is formed for supporting the curved central reflection portion 20 to be firmly fixed to the support portion 12. The support bars 121 may be installed at an interval of 60 degree angles on the outer circumferential surface of the support portion 12, but the angle may be variously changed.

In other words, six support bars 121 may be installed on the outer circumferential surface of the support portion 12. At one end of such a support bar 121, a tap is formed in a hole into which a bolt is inserted, so that the curved central reflection portion 20 may be coupled to the hole with bolts and nuts.

At this time, when the support bar 121 is assembled by being located in the center portion of the curved central reflection portion 20, a bottom surface of the curved central reflection portion 20 contacts the top end surface of the base portion 10 to form a fixed shape. Each of the curved central reflection portions 20 is in contact with the base portion 10, thereby eliminating the shaking of the support portion 12, achieving a balance, and ensuring the stability of the equipment.

The curved central reflection portion 20 is fixed by the support bar 121 and may be installed in a vertical direction from the base portion 10.

The curved central reflection portion 20 has one central ultraviolet lamp 30 installed on one side surface and reflects the ultraviolet rays output from the central ultraviolet lamp 30 on the curved reflection portion. That is, the curved central reflection portion 20 is a curved reflective lampshade that increases and overlaps the projection angle of the beam output from the central ultraviolet lamp 30, so that the ultraviolet rays emitted from the lamp maximally increase the sterilization effect.

The curved central reflection portion 20 may have the one side and the other side that are inclined, at the same angle, from the support portion 12 taking the middle of the length as a center, thereby expandingly reflecting the ultraviolet rays output from the central ultraviolet lamp 30 at a wide angle. At this time, the curved central reflection portion 20 may be formed in a curved shape inclined at an acute angle from the support portion 12.

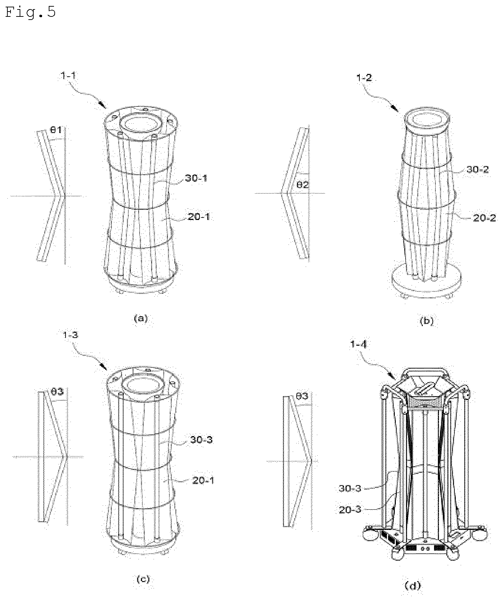

For example, in FIG. 5, each curved central reflection portion 20 may have the one end and an opposite end curved θ1 degree angles toward the support portion 12, whereby the cross section of the curved central reflection portions 20 may be formed in a shape being a part of a trophy type, or each curved central reflection portion 20 may have the one end and the opposite end curved θ2 degree angles from the support portion 12, whereby the cross section of the curved central reflection portions 20 may be formed in a shape being a part of a diamond type.

Contents for the operation and operation effect of the central ultraviolet lamp according to the curved shape of the curved central reflection portion 20 will be described together in FIGS. 5 to 7.

The handle portion 40 that may be gripped by a user to move the ultraviolet curved type sterilizer 1 may be installed outside the curved central reflection portion 20.

The handle portion 40 is configured to include a vertical stick 42 fixed on the top end surface of the base portion 10, a gripping portion 41 connecting the vertical sticks 42, and a horizontal stick 43 connecting the gripping portion 41 and the gripping portion 41. At this time, the handle portion 40 may be formed to be no less than the height of the central ultraviolet lamp.

Due to the handle portion 40, the user may move the ultraviolet curved type sterilizer 1 in a comfortable posture without bending at the waist.

In addition, the handle portion 40, where the central ultraviolet lamp 30, the curved central reflection portion 20, the mesh net cylinder 50, and the support bar 121 are located in sequence at the inner side thereof, also serves to protect the equipment.

A mesh net cylinder 50 may be installed between the curved central reflection portion 20 and the support portion 12. The mesh net cylinder 50 is formed in a hollow shape, thereby accommodating the support portion 12 therein, so that the support portion 12 is not visible from the outside, protecting the support portion 12 and the internal wiring, and facilitating waste heat discharge.

In addition, a wooden panel 51 formed by kneading wood flour, acetone, vegetable oil, and other additives may be placed on a top end portion of the mesh net cylinder 50. The wooden panel 51 is formed in a donut shape having a size no greater than the diameter of the mesh net cylinder and is fixed to the top end of the mesh net cylinder 50. The wooden panel 51 allows the upper end reflection portion 60 to be stably fixed to one side surface.

In addition, a plurality of perforated holes is formed on an outer circumferential surface of the mesh net cylinder 50, whereby the curved central reflection portion 20 on which the central ultraviolet lamp 30 is installed may be allowed to connect to the support bar 121 through the perforated hole.

The central ultraviolet lamp 30 emits ultraviolet rays. In the present specification, the central ultraviolet lamp 30 is a lamp that emits ultraviolet rays, that is, UV-C, of a wavelength range of 200 nm to 280 nm having high sterilizing power among ultraviolet rays. In particular, a lamp with a wavelength of 254 nm, which is a wavelength band having the best sterilization effect, is used.

The central ultraviolet lamp 30 may be formed of a quartz glass material, thereby being deformed in correspondence to the curved shape of the curved central reflection portion 20. The central ultraviolet lamp 30 of the ultraviolet curved type sterilizer 1 according to an embodiment of the present invention is a curved type lamp. However, the central ultraviolet lamp 30 is not limited to the curved type shape and may also be formed in a straight type shape.

As shown in FIG. 3, the central ultraviolet lamp 30 may be fixed to the central reflection unit 20 by means of holders 31, installed to one end and the opposite end, respectively, of the central ultraviolet lamp 30, and holder angles 32 assembled to the holders 31, respectively.

Each of the holders 31 serves to fix the central ultraviolet lamp 30 to the curved central reflection portion 20 and also serves to protect a connector of the central ultraviolet lamp 30, electric wires extended from the central ultraviolet lamp 30, and the like from the reflected rays of the ultraviolet rays output from the central ultraviolet lamp 30.

Each of the holder angles 32 is screwed to an associated holder 31 and may be adjusted by fixing the position by tightening, loosening, and the like of the screw so that the central ultraviolet lamp 30 made of a shape of a straight type or a curved type fits the shape of the holders. In other words, the holder angles 32 and the holders 31 are connected to be able to adjust the position angle of the holders 31.

Therefore, after the angle of each of the holders 31 is adjusted from one direction to another by loosening the screw fastened between the holder angle 32 and the holder 31, the position angle of the holder 31 may be adjusted while tightening the screw again. In particular, according to the application of the curved type lamp, the external shape of the ultraviolet curved type sterilizer 1 may also be changed.

As shown in FIG. 4(a), the ultraviolet curved type sterilizer 1 projects the ultraviolet rays up to and down on an area of one central ultraviolet lamp 30 by being located in a specific space, thereby having an overlapping range (only a part of areas is displayed). As shown in FIG. 4 (b), provided the overlapping areas of all the central ultraviolet lamps 30 are entirely displayed, it shows that the ultraviolet rays may be projected on every corner including in all directions of 360 degree angles and up to the bottom.

In particular, the ultraviolet curved type sterilizer 1 may project the ultraviolet rays to the ceiling surface of a specific space by means of the upper end reflection portion 60 and the upper end ultraviolet lamp 70, wherein the upper end reflection portion 60 is fixedly installed at the top end of the support portion 12, and the upper end ultraviolet lamp 70 projects the ultraviolet rays toward the outer side by being installed in an accommodating space of the upper end reflection portion 60. In addition, the ultraviolet curved type sterilizer 1 may project the ultraviolet rays up to the bottom, wherein the ultraviolet rays from one central ultraviolet lamp 30a and another central ultraviolet lamp 30b adjacent to each other are partially overlapped due to the curved central reflection portion 20.

At this time, the ultraviolet curved type sterilizer 1 introduces air thereinto through the vents 14 formed on one side surface of the base portion 10 and discharges heat generated from the power supply device 11 to the outside through the support portion 12 serving as a chimney, thereby allowing the power supply device 11 to be stably driven. Due to this structure, the central ultraviolet lamp 30 and the upper end ultraviolet lamp 70 are capable of being safely operated.

In addition, due to the motion detection sensors 13 configured to detect movement of objects and installed on one side surface of the base portion 10 at regular intervals, the ultraviolet curved type sterilizer 1 may control the central ultraviolet lamp 30 and the upper ultraviolet lamp 70 not to emit the ultraviolet rays when the motion of an object or of a person is sensed.

For example, when the central ultraviolet lamp 30 and the upper ultraviolet lamp 70 are emitting ultraviolet rays, and the motion detection sensors 13 detect a person's movement, the motion detection sensors 13 transmit signals to the controller 18. Thereafter, the controller 18 controls the power supply device 11 not to operate and may cut off the power supply so that the ultraviolet rays are not emitted from the central ultraviolet lamp 30 and the upper ultraviolet lamp 70.

Hereinafter, ultraviolet curved type sterilizer s for other embodiments of the present invention will be described with reference to FIGS. 5 to 7.

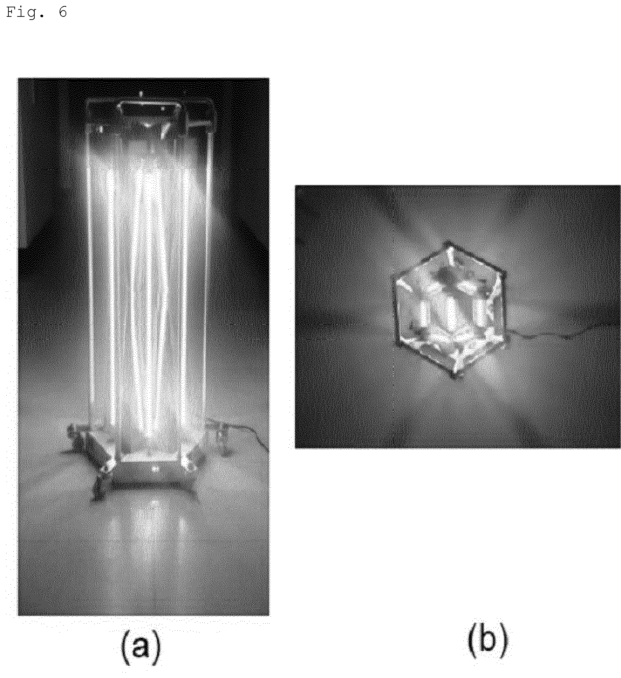

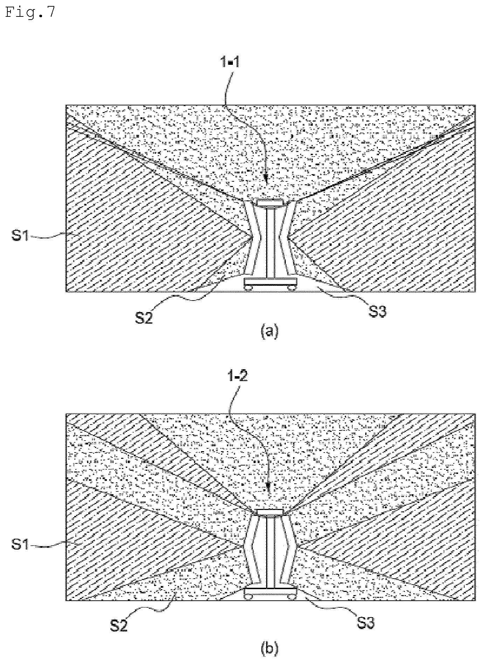

FIG. 5 shows perspective views of ultraviolet curved type sterilizer s for other embodiments of the present invention, and FIG. 6 shows photographs illustrating a front surface state and a top end surface state of FIG. 5(d). In addition, FIG. 7 shows views illustrating an overlapped distribution state of ultraviolet rays projected from the ultraviolet curved type sterilizer of FIG. 5.

First, FIG. 5(a) is a curved-lamp trophy type 1-1, FIG. 5(b) is a curved-lamp diamond type 1-2, FIG. 5 (c) is a straight-lamp trophy type 1-3, and FIG. 5(d) is a straight-lamp hexagonal trophy type 1-4. In addition, FIG. 6(a) shows a photograph illustrating the front surface state of FIG. 5(d), and FIG. 6(b) shows a photograph illustrating the top end surface state of FIG. 5(d).

FIG. 7 shows views illustrating an overlapped distribution state of ultraviolet rays projected from the ultraviolet curved type sterilizer of FIG. 5. The trophy and diamond types have more areas having the ultraviolet rays being overlapped than existing products. In particular, the trophy type forms many areas having the ultraviolet rays being overlapped and may exhibit a sterilization effect higher than other products.

The ultraviolet curved type sterilizer s for the other embodiments of the present invention are substantially the same as the ultraviolet curved type sterilizer for the one embodiment of the present invention, except for the central ultraviolet lamp and the curved central reflection portion.

Therefore, in order to simplify the description of the other embodiments of the present invention, descriptions of elements being overlapped with the one embodiment of the present invention will be omitted, and only elements having differences will be described.

A ultraviolet curved type sterilizer 1-1 shown in FIG. 5(a) includes a central ultraviolet lamp 30-1 curved in correspondence to a curved shape of a central reflection portion 20.

A central ultraviolet lamp 30-1 scatters the ultraviolet rays at a wide angle by means of the curved central reflection portion 20 and, as shown in FIG. 7(a), may overlap the ultraviolet rays on a wide area and form a wide area S1 with the ultraviolet rays being overlapped.

The area S1 with the ultraviolet rays being overlapped partially overlaps with an area with the ultraviolet rays of an upper end ultraviolet lamp 70 and may enhance a sterilizing effect for corner portions of the ceiling.

An ultraviolet curved sterilizer 1-2 shown in FIG. 5(b) may, differently from the curved shape of the curved central reflection portion 20 shown in FIG. 5(a), become a shape, that is, a part of the diamond type, in which one end and an opposite end of the curved central reflection part 20-2 are inclined by θ2 degree angles in an inner direction of the support portion 12. At this time, θ2 may be the same degree angles as θ1 described above.

As shown in FIG. 7(b), due to a shape protruding outward, a central ultraviolet lamp 30-2 has a drawback that an area being overlapped with ultraviolet rays between a plurality of the central ultraviolet lamps 30-2 is somewhat narrower compared with the area of the trophy type. However, an area being overlapped with ultraviolet rays on a ceiling portion is somewhat increased for the central ultraviolet lamp 30-2. As a result, as the area S1 being overlapped is present in the same manner, both of the above schemes are structures that may intensively project the ultraviolet rays on the area that may be most contacted by human hands in real life.

A ultraviolet curved type sterilizer 1-3 shown in FIG. 5(c) is an example in which a conventional straight type central ultraviolet lamp 30 is applied. FIG. 5(c) is a trophy type consisting of the straight type lamps. In the trophy type, the shape of the lamp may be variously modified.

A ultraviolet curved type sterilizer 1-4 shown in FIG. 5(d) is an example in which a conventional straight type central ultraviolet lamp 30 is applied. FIG. 5(d) is a hexagonal (arbitrarily shaped) trophy type that consists of straight type lamps, thereby being allowed to be modified in various directions.

FIGS. 6(a) and 6(b) are front surface and top end surface photographs, respectively, in which the ultraviolet rays are distributed and overlapped when the ultraviolet curved type sterilizer 1-4 to which the straight type lamps of FIG. 5(d) are applied is operated.

Here, looking at the front surface photograph of FIG. 6(a), it may be seen that as if three lamps appear to project instead of one light source due to a reflector, and the rays radiate by being overlapped. Looking at the top end surface photograph of FIG. 6(b), areas having the ultraviolet rays being overlapped described above appear clearly on the bottom surface.

On the other hand, existing products have disadvantages that it is difficult to sterilize the corners of the floor including the ceiling and that it is necessary to be used for a long time to sterilize areas which people's hands most touch, with a structure having no areas with the ultraviolet rays being overlapped.

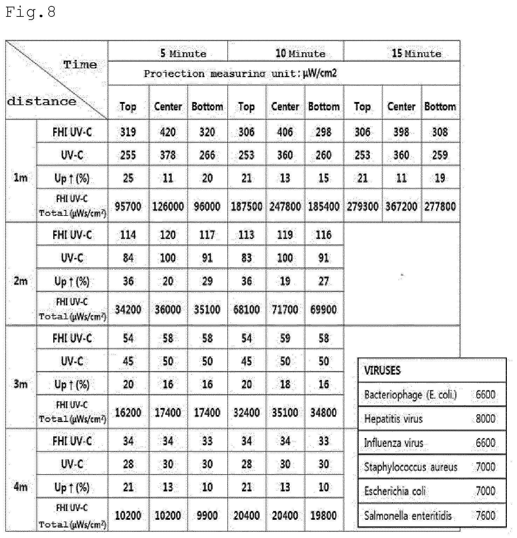

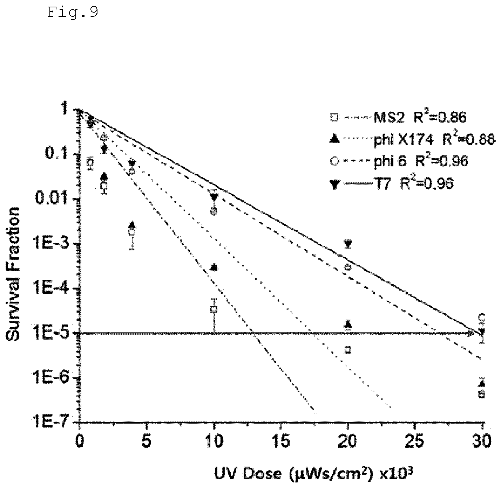

Hereinafter, with reference to data of FIGS. 8 and 9, data for a projection amount of the ultraviolet curved type sterilizer of the present invention and a projection amount of the conventional ultraviolet sterilizer will be comparatively analyzed to describe the sterilization effect of the ultraviolet curved type sterilizer of the present invention in detail.

FIG. 8 is data showing the projection amount of the ultraviolet curved type sterilizer of the present invention and the projection amount of the conventional ultraviolet sterilizer, and FIG. 9 is material data demonstrating sterilization performance of the ultraviolet curved type sterilizer of the present invention.

The FHI UV-C described in the data is a ultraviolet curved type sterilizer according to one embodiment and the other embodiments of the present invention. In addition, UV-C is a conventional ultraviolet sterilizer.

The present experimental data is the result of experiments with 2 straight type lamps.

When separated 1 m from the projection surface, the ultraviolet curved type sterilizer (FHI UV-C) of the present invention may project the ultraviolet rays of about 319 µW per 1 cm² of a top surface, about 420 µW per 1 cm² of the wall surface, and about 320 µW per 1 cm² of the bottom surface. At this time, the straight type central ultraviolet lamp 30 of the ultraviolet curved type sterilizer (FHI UV-C) is a lamp that outputs 127 Watts by being formed in a diameter of 20 mm and a length of 1 m.

The ultraviolet curved type sterilizer (FHI UV-C), which includes two straight type central ultraviolet lamps 30 provided with this shape and capability, may project the ultraviolet rays having a total projection amount of 95700 µWs/cm² on the top surface, 126000 µWs/cm² on the wall surface, and 96000 µWs/cm² on the bottom surface, for 5 minutes.

In addition, when the ultraviolet rays are projected more for another 5 minutes, 187500 µWs/cm² on the top surface, 247800 µWs/cm² on the wall surface, and 185400 µWs/cm² on the bottom surface may be projected, and when the ultraviolet rays are projected more for still another 5 minutes, 279300 µWs/cm² on the top surface, 367200 µWs/cm² on the wall surface, and 277800 µWs/cm² on the bottom surface may be projected. Here, when six central ultraviolet lamps are used, values higher than the values enumerated above may be obtained.

On the other hand, the conventional ultraviolet sterilizer (UV-C), in which the same lamp as the central ultraviolet lamp is installed, may project the ultraviolet rays of about 255 µW per 1 cm² of the top surface, about 378 µW per 1 cm² of the wall surface, and about 266uW per 1 cm² of the bottom surface when separated 1 m from the projection surface.

As such, by the conventional ultraviolet sterilizer (UV-C), 76500 µWs/cm² on the top surface, 113400 µWs/cm² on the wall surface, and 79800 µWs/cm² on the bottom surface may be projected for 5 minutes; 152400 µWs/cm² on the top surface, 221400 µWs/cm² on the wall surface, and 157800 µWs/cm² on the bottom surface may be projected when the ultraviolet rays are projected more for another 5 minutes; and 228300 µWs/cm² on the top surface, 329400 µWs/cm² on the wall surface, and 235500 µWs/cm² on the bottom surface may be projected when the ultraviolet rays are projected more for still another 10 minutes.

The ultraviolet curved type sterilizer (FHI UV-C) of the present invention may improve an amount of the ultraviolet projection at least 20% on the top surface, at least 10% on the wall surface, and at least 15% on the bottom surface, compared to the conventional ultraviolet sterilizer (UV-C). When six central ultraviolet lamps 30 are used, efficiencies higher than the efficiencies enumerated above may be obtained.

Such ultraviolet curved type sterilizer (FHI UV-C) may completely and quickly remove Baoteriophage being sterilized at 6600 µWs/cm², hepatitis virus being sterilized at 8000 µWs/cm², and influenza virus being sterilized at 6600 µWs/cm², due to the improved ultraviolet projection amount.

FIG. 9 is material data demonstrating the sterilization performance of the ultraviolet curved type sterilizer of the present invention.

The material data of FIG. 9 is data from experiments proceeded in a state of Room Humid (RH) 55%, wherein ultraviolet rays of 253.7 nm wavelength with an intensity of 120 µW/cm² are projected with a UV-C lamp for about 5 minutes in a small chamber of 0.27 m x 0.30 m x 0.3 m. The virus of each sample described in FIG. 9 is a representative one of several virus types.

As may be seen from a graph of FIG. 9, when an amount of projection is as much as an amount of 30,000 µWs/cm² on the X-axis, all viruses show a survival rate of no greater than 0.001% on the Y-axis. This is a result obtained at a distance of 0.3m, but compared to the FHI UV-C of FIG. 8, it is similar to the amount being projected for 15 minutes at a distance of 4m and to the amount being projected for about 3 minutes at a distance of 1m.

In other words, it means that all types of viruses may not survive at a maximum radius of 4m, in a short time.
Although the embodiments of the present invention have been described with reference to the accompanying drawings, those skilled in the art to which the present invention pertains will understand that the embodiments may be implemented in other specific forms without departing from the scope of protection defined by the claims Therefore, the embodiments described above are to be understood in all respects as illustrative and not restrictive.

## Claims

1. A ultraviolet curved type sterilizer (1), the sterilizer comprising:
a base portion (10) having a space accommodating a power supply device (11) therein and a support portion (12) formed on a top end portion thereof in a perpendicular direction to a bottom floor;
a plurality of curved central reflection portions (20) each fixed to the support portion (12) by having one end and an opposite end inclined at the same angle from the support portion (12) taking a middle of length as a center;
a central ultraviolet lamp (30) installed to project ultraviolet rays to the curved central reflection portion (20) toward an outer side, the central ultraviolet lamp (30) being formed of a quartz glass material;
an upper end reflection portion (60) formed with an accommodating space and fixedly installed at a top end of the support portion (12); and
an upper end ultraviolet lamp (70) installed in an accommodating space of the upper end reflection portion (60) and configured to project the ultraviolet rays toward an outer side,
**characterised in that**,
the sterilizer (1) comprises holder angles (32) and holders (31) installed at each of the opposite ends of the central ultraviolet lamp (30), each ^holder angle (32) having one side connected to one of the holders (32) and an opposite side connected to one end of the opposite ends of the curved central reflection portion (20), thereby allowing the holders (31) to be angle-adjusted from the curved central reflection portion (20), and when the central ultraviolet lamp (30) is assembled to the holder angle (32),
the central ultraviolet lamp (30) is able to be used by being modified in a '<' shape or a '>' shape.

2. The sterilizer of claim 1, wherein the plurality of curved central reflection portion (20) is installed at regular intervals on the support portion (12) so that the ultraviolet rays projected by the central ultraviolet lamp (30) installed on an outer side are allowed to be partially overlapped.

3. The sterilizer of claim 2, wherein the curved central reflection portion (20) is inclined at an acute angle from the support portion (12), the central ultraviolet lamp (30) corresponds to the inclination of the curved central reflection portion (20), and the central ultraviolet lamp (30) has the shape of a straight type or a curved type.

4. The sterilizer of claim 1, wherein the base portion (10) is provided with motion detection sensors (13) on a side surface thereof at regular intervals and configured to detect a movement of an object, and each of the base portion (10) and the support portion (12) includes a vent (14) configured to dissipate heat generated from the power supply device (11) to the outside.

5. The sterilizer of claim 4, wherein the motion detection sensors (13) are connected to the power supply device (11), so the sterilizer enables the power supply device (11) to be turned off when the motion detection sensors (13) detect a motion and enables the power supply device (11) to be turned on even when the motion detection sensors (13) are off.

## Patentansprüche

1. Gekrümmter Ultraviolett-Sterilisator (1), der Sterilisator Folgendes umfassend:
einen Basisabschnitt (10) mit einem Raum, der eine Stromversorgungsvorrichtung (11) aufnimmt, und einen Trägerabschnitt (12), der an einem oberen Endabschnitt davon senkrecht zu einem unteren Bodenbereich ausgebildet ist;
eine Vielzahl von gekrümmten zentralen Reflexionsabschnitten (20), die jeweils an dem Trägerabschnitt (12) befestigt sind, indem sie ein Ende und ein gegenüberliegendes Ende aufweisen, das in demselben Winkel von dem Trägerabschnitt (12) geneigt ist und bei dem eine Mitte der Länge das Zentrum darstellt;
eine zentrale Ultraviolett-Lampe (30), die so angebracht ist, dass sie Ultraviolettstrahlen auf den gekrümmten zentralen Reflexionsabschnitt (20) in Richtung einer Außenseite projiziert, wobei die zentrale Ultraviolett-Lampe (30) aus einem Quarzglasmaterial gebildet ist;
einen Reflexionsabschnitt am oberen Ende (60), der mit einem Aufnahmeraum ausgebildet ist und fest an einem oberen Ende des Trägerabschnitts (12) angebracht ist; und
eine Ultraviolett-Lampe am oberen Ende (70), die in einem Aufnahmeraum des Reflexionsabschnitt am oberen Ende (60) angebracht und so konfiguriert ist, dass sie die Ultraviolettstrahlen in Richtung einer Außenseite projiziert,
**dadurch gekennzeichnet, dass**
der Sterilisator (1) Halterungswinkel (32) und Halterungen (31) umfasst, die an jedem der einander gegenüberliegenden Enden der zentralen Ultraviolett-Lampe (30) angebracht sind, wobei jeder Halterungswinkel (32) eine Seite aufweist, die mit einer der Halterungen (32) verbunden ist, und eine gegenüberliegende Seite, die mit einem Ende der gegenüberliegenden Enden des gekrümmten zentralen Reflexionsabschnitts (20) verbunden ist, wodurch die Halterungen (31) von dem gekrümmten zentralen Reflexionsabschnitt (20) aus winkelverstellt werden können, und wenn die zentrale Ultraviolett-Lampe (30) an dem Halterungswinkel (32) montiert ist, kann die zentrale Ultraviolett-Lampe (30) verwendet werden, indem sie in eine '<'-Form oder eine '>'-Form gebracht wird.

2. Sterilisator nach Anspruch 1, wobei die Vielzahl von gekrümmten zentralen Reflexionsabschnitten (20) in regelmäßigen Abständen auf dem Trägerabschnitt (12) angebracht sind, so dass die von der auf einer Außenseite angebrachten zentralen Ultraviolett-Lampe (30) projizierten Ultraviolettstrahlen sich teilweise überlappen können.

3. Sterilisator nach Anspruch 2, wobei der gekrümmte zentrale Reflexionsabschnitt (20) in einem spitzen Winkel zum Trägerabschnitt (12) geneigt ist, die zentrale Ultraviolett-Lampe (30) der Neigung des gekrümmten zentralen Reflexionsabschnitts (20) entspricht und die zentrale Ultraviolett-Lampe (30) eine gerade oder gekrümmte Form hat.

4. Sterilisator nach Anspruch 1, wobei der Basisabschnitt (10) mit Bewegungserkennungssensoren (13) auf einer seiner Seitenflächen in regelmäßigen Abständen versehen ist, die so konfiguriert sind, dass sie eine Bewegung eines Objekts erfassen, und sowohl der Basisabschnitt (10) als auch der Trägerabschnitt (12) eine Entlüftung (14) enthält, die so konfiguriert ist, dass sie von der Stromversorgungsvorrichtung (11) erzeugte Wärme nach außen ableitet.

5. Sterilisator nach Anspruch 4, wobei die Bewegungserkennungssensoren (13) so mit der Stromversorgungsvorrichtung (11) verbunden sind, dass der Sterilisator es ermöglicht, die Stromversorgungsvorrichtung (11) auszuschalten, wenn die Bewegungserkennungssensoren (13) eine Bewegung erkennen, und es ermöglicht, die Stromversorgungsvorrichtung (11) einzuschalten, auch wenn die Bewegungserkennungssensoren (13) ausgeschaltet sind.

## Revendications

1. Un stérilisateur (1) à ultraviolets, de type incurvé, le stérilisateur comprenant :
une partie de base (10) ayant un espace logeant un dispositif d'alimentation électrique (11) et une partie de support (12) formée sur une partie d'extrémité supérieure de celle-ci dans une direction perpendiculaire à un plancher inférieur ;
une pluralité de parties de réflexion centrales incurvées (20) fixées chacune à la partie de support (12) en ayant une première extrémité et une extrémité opposée inclinées d'un même angle depuis la partie de support (12) en prenant comme centre un milieu de la longueur ;
une lampe à ultraviolets centrale (30) installée de façon à projeter des rayons ultraviolets vers la partie de réflexion centrale incurvée (20) vers un côté extérieur, la lampe à ultraviolets centrale (30) étant formée d'un matériau en verre de quartz ;
une partie de réflexion d'extrémité supérieure (60) formée avec un espace de logement et installée de manière fixe à une extrémité supérieure de la partie de support (12) ; et
une lampe à ultraviolets d'extrémité supérieure (70) installée dans un espace de logement de la partie de réflexion d'extrémité supérieure (60) et configurée pour projeter les rayons ultraviolets vers un côté extérieur,
**caractérisé en ce que**
le stérilisateur (7) comprend des organes de support (32) et des supports (31) installés à chacune des extrémités opposées de la lampe à ultraviolets centrale (30), chaque organe de support (32) ayant un côté relié à l'un des supports (32) et un côté opposé relié à une extrémité parmi les extrémités opposées de la partie de réflexion centrale incurvée (20), permettant ainsi aux supports (31) d'être réglés en termes d'angle à partir de la partie de réflexion centrale incurvée (20), et, lorsque la lampe à ultraviolets centrale (30) est assemblée à l'organe de support (32), la lampe à ultraviolets centrale (30) peut être utilisée en étant modifiée selon une forme en "<" ou une forme en ">".

2. Le stérilisateur selon la revendication 1, dans lequel les parties de réflexion de la pluralité de parties de réflexion centrales incurvées (20) sont installées à intervalles réguliers sur la partie de support (12) de sorte que les rayons ultraviolets projetés par la lampe à ultraviolets centrale (30) installée sur un côté extérieur puissent être partiellement en chevauchement.

3. Le stérilisateur selon la revendication 2, dans lequel la partie de réflexion centrale incurvée (20) est inclinée selon un angle aigu par rapport à la partie de support (12), la lampe à ultraviolets centrale (30) correspond à l'inclinaison de la partie de réflexion centrale incurvée (20), et la lampe à ultraviolets centrale (30) a une forme selon un type droit ou selon un type incurvé.

4. Le stérilisateur selon la revendication 1, dans lequel la partie de base (10) est pourvue de capteurs de détection de mouvement (13) sur une surface latérale d'elle-même à intervalles réguliers et configurés pour détecter un mouvement d'un objet, et chacune parmi la partie de base (10) et la partie de support (12) comprend un évent (14) configuré pour dissiper la chaleur générée par le dispositif d'alimentation (11) vers l'extérieur.

5. Le stérilisateur selon la revendication 4, dans lequel les capteurs de détection de mouvement (13) sont connectés au dispositif d'alimentation (11), de sorte que le stérilisateur permet au dispositif d'alimentation (11) d'être mis hors fonctionnement lorsque les capteurs de détection de mouvement (13) détectent un mouvement et permet au dispositif d'alimentation (11) d'être mis en fonctionnement même lorsque les capteurs de détection de mouvement (13) sont mis hors fonctionnement.
